(19) Europäisches Patentamt
European Patent Office
Office européen des brevets

(11) Veröffentlichungsnummer : **0 180 650**
**B1**

(12) **EUROPÄISCHE PATENTSCHRIFT**

(45) Veröffentlichungstag der Patentschrift : **25.05.88**

(51) Int. Cl.⁴ : **C 07 C155/10**, C 07 D295/20

(21) Anmeldenummer : **84112964.6**

(22) Anmeldetag : **27.10.84**

(54) **Verfahren zur Herstellung von Thiurampolysulfiden.**

(43) Veröffentlichungstag der Anmeldung : **14.05.86 Patentblatt 86/20**

(45) Bekanntmachung des Hinweises auf die Patenterteilung : **25.05.88 Patentblatt 88/21**

(84) Benannte Vertragsstaaten : **BE DE FR GB IT**

(56) Entgegenhaltungen :
**DE-A- 2 725 166**
**GB-A- 2 093 029**
**US-A- 2 794 021**

(73) Patentinhaber : **Akzo GmbH**
**Postfach 10 01 49 Kasinostrasse 19-23**
**D-5600 Wuppertal-1 (DE)**

(72) Erfinder : **Bergfeld, Manfred, Dr. Dipl.-Chem.**
**August Pfeffer Strasse 6**
**D-8765 Erlenbach (DE)**
Erfinder : **Eisenhuth, Ludwig, Dr. Dipl.-Chem.**
**Lautenhofstrasse 44**
**D-8753 Obernburg (DE)**
Erfinder : **Zengel, Hans-Georg, Dr. Dipl.-Chem.**
**Nordring 6**
**D-8751 Kleinwallstadt (DE)**

**EP 0 180 650 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von Thiurampolysulfiden aus sekundären Aminen, Schwefelkohlenstoff und Schwefel in Gegenwart eines Oxidationsmittels.

Thiurampolysulfide werden vor allem als Schwefeldonatoren und Beschleuniger bei der Vulkanisation von Kautschuk verwendet. Diese Substanzen werden z. B. unter dem Namen Thiuramtetrasulfid (Tetramethyl- oder Dipentamethylen-thiuramtetrasulfid) oder Thiuramhexasulfid (Dipentamethylenthiuramhexasulfid) in den Handel gebracht. Die chemische Konstitution dieser Produkte ist bislang nicht genau bekannt, da Analysenmethoden zur Unterscheidung zwischen Mischungen verschiedener Polysulfide und Mischungen aus Polysulfiden und Schwefel fehlen. Außerdem handelt es sich bei Thiurampolysulfiden um empfindliche Substanzen, die — besonders in gelöster Form — zur Abspaltung von Schwefel neigen.

Diese Problematik wird z. B. in der DE-A-27 25 166 am Beispiel des als Tetramethylthiuramtetrasulfid bezeichneten Materials ausführlich beschrieben. Durch den Einsatz moderner Analysenmethoden wie z. B. Hochdruckflüssigkeitschromatographie (HPLC) und Gelpermeationschromatographie (GPC) kann man qualitativ aufzeigen, daß bei den oben genannten Handelsprodukten keine einheitlichen Verbindungen vorliegen, sondern Gemische aus mehreren Polysulfiden und freiem Schwefel mit je nach Herstellungsmethode variierender quantitativer Zusammensetzung.

Im allgemeinen gehen die Verfahren zur Herstellung von Thiurampolysulfiden von den entsprechenden Dithiocarbamaten aus, die üblicherweise aus einem sekundären Amin, Schwefelkohlenstoff und einem Alkali- oder Erdalkalihydroxid hergestellt werden.

So beschreiben die US-PS 1 681 717 und 1 780 545 ein Verfahren zur Herstellung von Thiurampolysulfiden durch Umsetzung von Dithiocarbamaten mit Schwefelchloriden nach folgender Reaktionsgleichung :

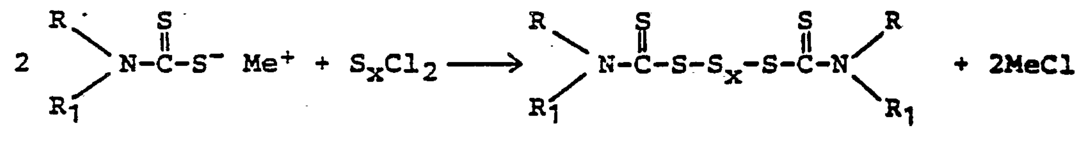

Hierbei werden jedoch nur geringe Ausbeuten erzielt. Ein verbessertes Verfahren zur Herstellung von Thiuramtetrasulfiden, insbesondere Dipentamethylenthiuramtetrasulfid, auf Basis obiger Reaktionsgleichung unter Verwendung von Schwefelmonochlorid, wird in der US-PS 2 414 014 beschrieben. Mit diesem Verfahren können bis zu 95 % Ausbeute erzielt werden.

Alle diese Verfahren haben jedoch die Nachteile gemeinsam, daß mit den korrosiven, übelriechenden Schwefelchloriden gearbeitet werden muß, und daß zusätzlich große Mengen unverwertbarer Salze als problematische Beiprodukte gebildet werden.

Ein Verfahren, das die zusätzliche Bildung von Natriumchlorid ausschließt, beschreibt die DE-A-27 25 166. Nach diesem Verfahren wird Dimethylammoniumdimethyldithiocarbamat mit Wasserstoffperoxid in Gegenwart von Schwefelkohlenstoff und Schwefel zum Tetramethylthiuramtetrasulfid umgesetzt.

In einer Variante dieses Verfahrens gemäß DE-A-27 25 166 wird das umzusetzende Dithiocarbamatsalz in einem vorgeschalteten Reaktionsschritt aus Dimethylamin und Schwefelkohlenstoff in Wasser gebildet, und es kann anschließend die hierbei erhaltene wäßrige Lösung des Dimethylammoniumdimethyldithiocarbamats mit Schwefel und Wasserstoffperoxid im selben Reaktionsgefäß weiter zum Tetramethylthiuramtetrasulfid umgesetzt werden. So wird gemäß Beispiel 1 der DE-A-27 25 166 ein Reaktionsgefäß mit Wasser, Dimethylamin und 2 Tropfen eines nichtionischen grenzflächenaktiven Mittels gefüllt, die Lösung bei 25 °C gerührt und Schwefelkohlenstoff während einer Zeitspanne von 14 Minuten zugesetzt, wobei die Temperatur auf 35 °C steigt. Der Schwefel wird nun in einer Portion zugesetzt, woran sich die Zugabe von Wasser anschließt. Dann wird Schwefelkohlenstoff der erhaltenen Aufschlämmung während einer Zeitspanne von 60 Minuten gleichzeitig mit Wasserstoffperoxid zugesetzt, wobei die Peroxidzugabe 2 Minuten nach Beginn der Schwefelkohlenstoffzugabe erfolgt. Das Endprodukt fällt schließlich nach Filtration in einer Ausbeute von 90 % an.

Dieses Verfahren stellt zwar eine Verbesserung gegenüber dem eingangs erwähnten Verfahren dar, ist allerdings in seiner Anwendung auf die Herstellung von Tetramethylthiuramtetrasulfid beschränkt. Weitere Nachteile sind das Erfordernis eines vergleichsweise teuren und wenig selektiven Oxidationsmittels (Wasserstoffperoxid), eines nichtionischen grenzflächenaktiven Mittels und die nicht quantitative Ausbeute.

Zur Herstellung von Thiuramsulfiden wurde kürzlich ein weniger aufwendiges Verfahren durch Umsetzung von sekundären Aminen und Schwefelkohlenstoff in Gegenwart eines tertiären Amins und eines Oxidationsmittels in der DE-A-31 05 587 beschrieben. Dieses Verfahren, das mit metallhaltigen Katalysatoren und Sauerstoff als Oxidationsmittel arbeitet, führt in hoher Ausbeute zu Thiuramdisulfiden.

Nach einem vereinfachten Verfahren zur Herstellung von Thiurampolysulfiden aus billigen Ausgangsverbindungen in hoher Ausbeute bestand weiterhin ein Bedürfnis.

Die hieraus resultierende Aufgabe konnte nun erfindungsgemäß gelöst werden durch ein Verfahren zur Herstellung von mit aliphatischen, araliphatischen, cycloaliphatischen und/oder aromatischen Kohlenwasserstoffresten substituierten Thiurampolysulfiden durch Umsetzung eines entsprechend substituierten sekundären Amins mit Schwefelkohlenstoff und Schwefel in einem Lösungsmittel und in Anwesenheit eines Oxidationsmittels, das dadurch gekennzeichnet ist, daß die Umsetzung in Gegenwart eines tertiären Amins bei Temperaturen von 0 bis 150 °C durchgeführt wird und als Oxidationsmittel Sauerstoff oder ein sauerstoffhaltiges Gas und ein metallhaltiger Katalysator verwendet werden.

Das erfindungsgemäße Verfahren eignet sich für die Herstellung einer Vielzahl sehr unterschiedlich substituierter Thiurampolysulfide mit unterschiedlichem Schwefelgehalt.

Setzt man nur ein einziges sekundäres Amin als Reaktionspartner ein, so erhält man ein Thiurampolysulfid, welches an beiden Stickstoffatomen dieselben Substituenten trägt. Setzt man zwei unterschiedliche sekundäre Amine als Reaktionspartner ein, so kann man je nach Verfahrensbedingungen (Basizitätsunterschiede der Amine, Molverhältnisse, etc.) Thiurampolysulfide mit zwei unterschiedlich substituierten Stickstoffatomen erhalten ; als Nebenprodukte können hierbei noch mehr oder weniger große Mengen der beiden symmetrisch substituierten Thiurampolysulfide entstehen. Die Länge der Schwefelbrücke in den Thiurampolysulfiden wird durch die eingesetzte Schwefelmenge festgelegt. Setzt man z. B. 1 Grammatom Schwefel auf 1 Mol sekundäres Amin ein, so erhält man ein Produkt mit einer im Mittel aus 4 Schwefelatomen bestehenden Schwefelbrücke (Tetrasulfid). Setzt man 2 Grammatom Schwefel pro Mol Amin ein, so wird man im Mittel ein Hexasulfid erhalten.

Für das Verfahren sind alle sekundären Amine geeignet. Ein derartiges sekundäres Amin wird dargestellt durch die Formel

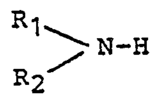

wobei $R_1$ und $R_2$ gleich oder verschieden sein können und jeweils $C_1$-$C_{18}$-Alkylreste wie z. B. Methyl, Äthyl, Propyl, n-Butyl, t-Butyl, Hexyl, Dodecyl und Octadecyl, Cycloalkylreste wie Cyclopentyl- und Cyclohexylrest und die durch Alkylgruppen substituierten Cyclopentyl- und Cyclohexylreste, durch Arylreste wie Phenyl und Naphthylreste substituierten $C_1$-$C_{18}$-Alkylreste sowie aromatische Reste wie Phenyl- und Naphthylreste und die durch Alkylgruppen substituierten Phenyl- und Naphthylreste und die durch Alkylgruppen substituierten Phenyl- und Naphthylreste darstellen. Die Substituenten des sekundären Amins können auch über ein gemeinsames Brückenglied miteinander verbunden sein. Beispiele für derartige Amine sind Piperidin, Pyrolidin, Morpholin und deren Derivate sowie andere Stickstoff-Heterocyclen.

Geeignete tertiäre Amine sind aliphatisch, cycloaliphatisch und/oder aromatisch substituierte Amine sowie Heterocyclen mit einem trisubstituierten Stickstoffatom. Bevorzugt werden tertiäre Amine mit einem pKa-Wert $> 8$ eingesetzt, beispielsweise Trimethylamin, Triäthylamin, Tri-n-propylamin, Tri-n-butylamin, n-Octyl-dimethylamin, Äthyldimethylamin, Propyldimethylamin, Butyldimethylamin, Tetramethyläthylendiamin, N-Methylpyrolidin, N-Dimethylamino-pyridin und 1.4-Diazabicyclo-(2.2.2)-octan.

Die Menge des tertiären Amins kann in weiten Grenzen variiert werden. Sie reicht von katalytischen bis stöchiometrischen Mengen und darüber hinaus bis zu Mengen, welche den Mengen des Lösungsmittels entsprechen oder sie sogar übertreffen. Das tertiäre Amin kann auch als Lösungsmittel fungieren.

Als Oxidationsmittel wird beim erfindungsgemäßen Verfahren Sauerstoff oder ein sauerstoffhaltiges Gas, insbesondere Luft, verwendet.

Der Schwefel kann fest, flüssig oder gelöst, z. B. in Schwefelkohlenstoff, zugegeben werden. Dabei wurde festgestellt, daß es sich bei dem erfindungsgemäßen Verfahren um eine völlig neuartige Reaktion handelt. Überraschenderweise wird nämlich bei dieser Reaktion eine wesentlich höhere Oxidationsgeschwindigkeit erzielt, als bei einer entsprechenden Reaktion ohne Schwefelzusatz, die zu einfachen Thiuramdisulfiden führt ; das bedeutet, der Schwefel übt einen stark beschleunigenden Effekt auf die Oxidation aus.

Das Lösungsmittel ist beim erfindungsgemäßen Verfahren nicht kritisch, und es kommen so verschiedene Substanzklassen wie aromatische Kohlenwasserstoffe, wie Benzol, Toluol, Xylol, Nitrobenzol, aliphatische Ester, Alkyläther, niedere Alkohole wie Methanol, Äthanol, Isopropanol, n-Propanol, n-Butanol, t-Butanol und Amylalkohol, weiter Chlorkohlenwasserstoffe, wie Dichlormethan, Chloroform, Dichloräthan, Trichloräthan, ferner aprotische Lösungsmittel wie Dimethylformamid, Acetonitril, Dimethylacetamid, Dimethylsulfoxid und Hexamethylphosphorsäuretriamid sowie Wasser oder ein Gemisch der genannten Lösungsmittel in Betracht.

Je nach eingesetztem sekundärem Amin können in reinem Wasser in einzelnen Fällen hohe Ausbeuten und Selektivitäten erzielt werden. Im allgemeinen ist die Reaktionsgeschwindigkeit in Wasser jedoch geringer als in den obengenannten nichtwäßrigen Lösungsmitteln. Vorzugsweise werden als Lösungsmittel aromatische Kohlenwasserstoffe, niedere Alkohole mit bis zu 6-C-Atomen, Gemische dieser Lösungsmittel oder Gemische dieser niederen Alkohle mit Wasser eingesetzt.

Als metallhaltige Katalysatoren eignen sich alle Metalle der Nebengruppen und deren Derivate, die zu einem leichten Valenzwechsel befähigt sind. Vorzugsweise werden als metallhaltige Katalysatoren Cer,

Mangan, Kupfer, Eisen, Kobalt, Molybdän oder Vanadin in elementarer Form oder als Salze, Oxide, Komplexe oder in Form ihrer organischen Verbindungen eingesetzt. Von den bevorzugten Metallen bzw. deren Derivaten besitzen Kupfer, Mangan und Cer, verglichen mit Eisen, Kobalt, Molybdän und Vanadin die höhere katalytische Wirksamkeit, jedoch sind auch die letztgenannten Metalle und ihre Derivate ausgezeichnete Katalysatoren für die Oxydation.

Elementares Kupfer kommt vorzugsweise als Kupferpulver zur Anwendung. Als Kupferverbindung werden alle ein- oder zweiwertigen anorganischen, organischen, einfachen oder komplexen Kupfersalze in Betracht gezogen. Beispiele geeigneter einwertiger Kupfersalze sind Kupfer-(I)-chlorid, -bromid und -jodid, Additionsverbindungen dieser Kupfer-(I)-halogenide mit Kohlenmonoxid, komplexe Kupfer-(I)-salze, wie die Alkalichlorcuprate, komplexe Ammoniakate des Kupfer-(I)-cyanids, z. B. Cyanocuprate, wie Kalium-tricyanocuprat(I), Doppelsalze mit Kupfer-(I)-rhodanid, Kupfer-(I)-acetat, Kupfer-(I)-sulfid und komplexe Doppelsulfide aus Kupfer-(I)-sulfid und Alkalipolysulfiden. Beispiele geeigneter Kupfer-(II)-salze sind Kupfer-(II)-chlorid, -bromid, -sulfid, -sulfat, -nitrat, -nitrit, -rhodanid, -cyanid, Cu-(II)-Salze von Carbonsäuren, wie Kupfer-(II)-acetat, Kupferdithiocarbamat sowie die komplexen Ammoniakate von Kupfer-(II)-salzen. Auch Kupfer-(I)-oxid ist sehr gut als Katalysator geeignet.

Beispiele geeigneter manganhaltiger Katalysatoren sind Manganpulver, Mangandioxid, Kaliumpermanganate, Manganacetat und die Mangandithiocarbamate sowie die übrigen den obengenannten Kupferverbindungen entsprechenden Manganderivate. Als Beispiele geeigneter Cer-Katalysatoren werden metallisches Cer, Cerdioxid, Cer-(III)-chlorid, Cer-(IV)-chlorid und die Cer-chlorokomplexsalze, Cernitrat und -nitratosalze, Cersulfat, Cercarbonat, Ceroxalat und die Cersulfide genannt.

Beispiele für Eisenkatalysatoren sind die bekannten Eisenoxide, Eisen-(II)- und Eisen-(III)-salze sowie die Komplexsalze.

Beispiele geeigneter Vanadinkatalysatoren sind die Vanadinoxide, -chloride und -sulfate sowie die bekannten Doppel- und Komplexsalze.

Geeignete Kobaltkatalysatoren sind die bekannten Kobaltoxide, Kobalt-(II)-salze und die Komplexsalze.

Schließlich seien als Beispiele geeigneter Molybdänkatalysatoren die Oxide, Chloride, Sulfide und Fluoride, ferner die Molybdate sowie die bekannten komplexen Acidosalze genannt.

Selbstverständlich können auch Gemische aus mehreren der genannten Katalysatoren eingesetzt werden.

Die erforderliche Menge des metallhaltigen Katalysators ist überraschend gering. Sie liegt vorzugsweise im Bereich von 0,01 bis 5 mMol, bezogen auf 1 Mol sekundäres Amin. Es können auch geringere Katalysatormengen zur Anwendung gelangen, jedoch müssen hierbei längere Reaktionszeiten in Kauf genommen werden. Höhere Mengen an Katalysatoren sind nicht empfehlenswert, weil dann die Gefahr besteht, daß der Katalysator ausfällt und das Reaktionsprodukt verunreinigt.

Das erfindungsgemäße Verfahren wird bei Temperaturen im Bereich von 0 bis 150 °C durchgeführt, vorzugsweise bei 20 bis 90 °C. Temperaturen oberhalb 90 °C erhöhen zwar die Raum/Zeitausbeute, werden jedoch aus sicherheitstechnischen Gründen weniger bevorzugt.

Vorzugsweise wird das erfindungsgemäße Verfahren bei Sauerstoffdrücken bzw. Sauerstoffpartialdrücken von wenigstens 0,1 bar durchgeführt. Wie zu erwarten, erhöht sich mit steigendem Druck die Reaktionsgeschwindigkeit. Aus sicherheitstechnischen Gründen wird ein Druckbereich von 1-10 bar bevorzugt.

Zur Durchführung des Verfahrens können die Reaktanten, Katalysator und Lösungsmittel in jeder beliebigen Reihenfolge zusammengegeben werden. Das sekundäre Amin und der Schwefelkohlenstoff werden dabei im allgemeinen ungefähr in stöchiometrischem Verhältnis eingesetzt (1 : 1) ; vorzugsweise wird der Schwefelkohlenstoff in einem leichten Überschuß (0,01 bis 0,2 Mol Überschuß) verwendet. Die einzusetzende Schwefelmenge kann je nach gewünschtem Endprodukt in weitem Bereich variiert werden. Bevorzugt wird ein Anteil von 1 bis 3 Grammatom Schwefel/Mol sekundäres Amin. Setzt man 1 Grammatom Schwefel pro Mol sekundäres Amin ein, so wird man im wesentlichen ein Thiuramtetrasulfid erhalten, bei 2 Grammatom Schwefel ein Thiuramhexasulfid und bei noch größeren Schwefelmengen werden entsprechend höhere Thiurampolysulfide gebildet. Besonders bevorzugt werden 1 bis 2 Grammatom Schwefel pro Mol sekundäres Amin eingesetzt.

Gemäß einer Ausführungsform der Erfindung werden das sekundäre Amin, der Schwefelkohlenstoff, der Schwefel, das tertiäre Amin und der metallhaltige Katalysator im Lösungsmittel aufgelöst bzw. suspendiert und in Anwesenheit von Sauerstoff bzw. eines sauerstoffhaltigen Gases zum entsprechenden Thiurampolysulfid umgesetzt. Ebensogut ist es möglich, das aus sekundärem Amin, Schwefelkohlenstoff und tertiärem Amin als Zwischenprodukt gebildete Dithiocarbamat zu isolieren und anschließend dieses Dithiocarbamat mit Schwefel in Anwesenheit von Sauerstoff bzw. eines sauerstoffhaltigen Gases und des metallhaltigen Katalysators umzusetzen. Weiterhin ist es auch möglich, die Reaktionspartner sekundäres Amin, Schwefelkohlenstoff und Schwefel während der Reaktion der Reaktionslösung zuzuführen.

Die Reaktionsdauer hängt von den Verfahrensbedingungen sowie von dem eingesetzten sekundären Amin ab und liegt im allgemeinen im Bereich von wenigen Minuten bis zu mehreren Stunden. Unter günstigen Bedingungen bezüglich Temperatur und Sauerstoffdruck beträgt sie wenige Minuten bis 1 Stunde.

Die Durchführung des erfindungsgemäßen Verfahrens erfolgt in einfacher Weise dadurch, daß der

Sauerstoff bzw. das sauerstoffhaltige Gas unter den angegebenen Druck- und Temperaturbedingungen auf das Reaktionsgemisch aufgepreßt oder in bzw. durch das Reaktionsgemisch geleitet wird. Das Ende der Reaktion (bei vollständigem Umsatz) läßt sich in einfacher Weise z. B. am Ende der Sauerstoffaufnahme erkennen.

In den meisten Fällen, wie z. B. bei den Tetramethylthiurampolysulfiden oder Dipentamethylenthiurampolysulfiden, fällt das Endprodukt sofort in fester Form aus dem Reaktionsgemisch aus und kann abfiltriert werden. In anderen Fällen erhält man das gewünschte Produkt durch Abkühlen oder Einengen des Reaktionsgemisches. Flüssige Produkte werden durch destillative oder extraktive Aufarbeitung in reiner Form erhalten.

Bei der technischen Durchführung des erfindungsgemäßen Verfahrens ist es vorteilhaft, die im wesentlichen aus Lösungsmittel, tertiärem Amin und metallhaltigem Katalysator bestehende Mutterlauge im Kreislauf zu führen, wobei es nicht erforderlich ist, stets frisches tertiäres Amin oder metallhaltigen Katalysator zuzusetzen. Beispielsweise können mehr als zehn Reaktionszyklen mit der gleichen Mutterlauge mit unverändert hoher Ausbeute durchgeführt werden, ohne daß ein Abfall der katalytischen Aktivität der Mutterlauge festgestellt wird.

Beim erfindungsgemäßen Verfahren können in den meisten Fällen praktisch quantitative Ausbeuten und Selektivitäten von mehr als 99 % erzielt werden. Die Produkte fallen in hoher Reinheit an und können in der Regel ohne Reinigung ihrer Bestimmung zugeführt werden. Bei entsprechendem Schwefelzusatz entsprechen die erhaltenen Produkte in ihrer chemischen Zusammensetzung den im Handel befindlichen Produkten (z. B. Tetramethyl- oder Dipentamethylenthiuramtetrasulfid oder Dipentamethylenthiuramhexasulfid).

Gegenüber dem bekannten zweistufigen Verfahren bei welchem zunächst die Dithiocarbamate synthetisiert werden, zeichnet sich das einstufige Verfahren durch seine Wirtschaftlichkeit und Umweltfreundlichkeit aus, da keine Hilfsstoffe verbraucht werden. Gegenüber dem aus der DE-A-27 25 166 bekannten einstufigen, aber lediglich auf die Herstellung von Tetramethylthiuramtetrasulfid beschränkten Verfahren besitzt das erfindungsgemäße Verfahren den Vorteil, daß in einfacher Reaktionsführung ein wesentlich preiswerteres Oxidationsmittel eingesetzt werden kann und daß praktisch quantitative Ausbeuten und hohe Selektivitäten erzielt werden.

Die folgenden Beispiele erläutern die Erfindung näher.

## Beispiel 1

In einem 1 l Glasautoklaven, der mit einem Doppelmantel zur Zirkulation einer Heizflüssigkeit, einem Thermometer, einem Druckmeßgerät und einer Rührvorrichtung ausgerüstet ist, werden zu einer Lösung von 34,06 g (0,4 mol) Piperidin, 20,2 g (0,2 mol) Triäthylamin und 4,9 mg ($0{,}02 \cdot 10^{-3}$ mol) Mangan-II-acetat-tetrahydrat in 280 ml Methanol 25,64 g (0,8 Grammatom) Schwefel und 31,2 g (0,41 mol) Schwefelkohlenstoff gegeben. Das Reaktionsgemisch wird auf 50 °C erwärmt, intensiv gerührt und mit 1,8 bar Sauerstoff beaufschlagt. Sofort wird ein Sauerstoffverbrauch registriert, und es bildet sich ein fast weißer Niederschlag. Nach 55 min. ist die Reaktion beendet (keine weitere Sauerstoffaufnahme). Das Piperidin ist vollständig umgesetzt. Der Niederschlag wird abfiltriert, mit Methanol gewaschen und getrocknet. Es fallen 87,8 g Produkt mit einem Schmelzbereich 124-127 °C an.

Dieses Produkt entspricht in seiner Zusammensetzung dem Dipentamethylenthiuramhexasulfid.

Analyse : Dipentamethylenthiuramhexasulfid $C_{12}H_{20}N_2S_8$

| | | | | |
|---|---|---|---|---|
| ber. : | C 32,11 | H 4,49 | N 6,24 | S 57,15 % |
| gef. : | C 32,2 | H 4,6 | N 5,9 | S 57,4 % |

Durch Hochdruckflüssigkeitschromatographie-Analyse wird nachgewiesen, daß der Gehalt an freiem Schwefel im Produkt und der Gehalt an Dipentamethylenthiuramdisulfid jeweils unter 1 % liegen.

Die Mutterlauge enthält noch weitere 1,2 g des Produktes, die durch Einengen oder starkes Abkühlen isoliert werden können ; auch das eingesetzte Triäthylamin ist unverändert in der Mutterlauge enthalten. Demnach beträgt die Gesamtausbeute 89,0 g, entsprechend 99,1 % d. Th.

Das nach diesem Verfahren hergestellte Produkt entspricht als Schwefeldonator den im Handel befindlichen, als Dipentamethylenthiuramtetrasulfid bezeichneten Produkten.

## Beispiel 2 (Vergleichsbeispiel)

Es wird wie in Beispiel 1 gearbeitet, aber ohne Schwefelzusatz. Die Oxidation die in diesem Fall zu Dipentamethylenthiuramdisulfid führt, erfolgt wesentlich langsamer. Nach 55 min. Reaktionszeit enthält das Reaktionsgemisch lediglich 11,2 g Dipentamethylenthiuramdisulfid (entsprechend 17,5 % d. Th.) mit einem Schmelzpunkt von 132 °C. Dieses Beispiel zeigt, daß der Schwefel die Reaktion stark beschleunigt.

## Beispiel 3

In der in Beispiel 1 beschriebenen Reaktionsapparatur werden 78,7 g (0,3 mol) Triäthylammoniumpentamethylendithiocarbamat, das z. B. durch Umsetzung von Triäthylamin, Piperidin und Schwefelkohlenstoff in alkoholischer Lösung hergestellt werden kann, in 300 ml Methanol und bei Anwesenheit von 6,1 mg ($0,025 \cdot 10^{-3}$ mol) Mangan-II-acetat-tetrahydrat mit 9,6 g (0,3 mol) Schwefel und Sauerstoff in der in Beispiel 1 beschriebenen Weise zur Reaktion gebracht. Die Reaktionstemperatur beträgt 50 °C, der Sauerstoffdruck 1,7 bar und die Reaktionszeit 70 min.

Dabei wird ein Produkt mit einem Schmelzbereich 123-128 °C erhalten, das in seiner Zusammensetzung dem Dipentamethylenthiuramtetrasulfid entspricht.

Analyse : Dipentamethylenthiuramtetrasulfid $C_{12}H_{20}N_2S_6$
ber. : C 37,46 % H 5,24 % N 7,28 % S 50,01 %
gef. : C 37,4 % H 5,1 % N 7,0 % S 50,2 %

Der Gehalt an freiem Schwefel liegt unter 1 % (HPLC-Analyse).
Die Ausbeute beträgt 56,9 g (98,7 % d. Th.).

Beispiel 4

Zur Herstellung von N-N'-Dimethyl-N,N'-diphenyl-thiurampolysulfid werden 32,15 g (0,3 mol) N-Methylanilin, 23,6 g (0,31 mol) Schwefelkohlenstoff, 31,37 g (0,31 mol) Triäthylamin und 9,6 g (0,3 Grammatom) Schwefel in 300 ml Methanol in der in Beispiel 1 angegebenen Weise mit Sauerstoff zur Reaktion gebracht. Als Katalysator werden $0,1 \cdot 10^{-3}$ mol Mangan-II-acetat verwendet, die Reaktionstemperatur beträgt 50 °C und der Sauerstoffdruck 1,8 bar. Nach 150 min. wird der Versuch abgestellt und der entstandene feine, leicht gelbliche Niederschlag abfiltriert, gewaschen und getrocknet. Das resultierende Produkt mit einem Schmelzbereich 160-170 °C entspricht in seiner Zusammensetzung dem N,N'-Dimethyl-N,N'-diphenylthiuramtetrasulfid.

Analyse : N,N'-Dimethyl-N,N'-diphenylthiuramtetrasulfid $C_{16}H_{16}N_2S_6$
ber. : C 44,8 % H 3,8 % N 6,5 % S 44,9 %
gef. : C 44,4 % H 3,6 % N 6,2 % S 45,5 %

Die Ausbeute beträgt 58,4 g (90,8 % d. Th.).

Beispiel 5 (Vergleichsbeispiel)

Es wird wie in Beispiel 4 gearbeitet, aber ohne den Zusatz von Triäthylamin. Unter diesen Bedingungen findet praktisch keine Sauerstoffaufnahme statt. Nach 150 min. Reaktionszeit werden die eingesetzten Stoffe unverändert vorgefunden.

Beispiel 6

In der in Beispiel 1 beschriebenen Reaktionsapparatur werden 34,8 g (0,4 mol) Morpholin, 31,2 g (0,41 mol) Schwefelkohlenstoff, 20,2 g (0,2 mol) Triäthylamin und 25,64 g (0,8 Grammatom) Schwefel in 300 ml Methanol in Gegenwart von $0,025 \cdot 10^{-3}$ mol Mangan-II-acetat mit Sauerstoff zur Reaktion gebracht. Die Reaktionstemperatur beträgt 50 °C und der Sauerstoffdruck 1,7 bar. Nach 270 min. wird der Versuch abgestellt. Der beigefarbene feine Niederschlag wird abfiltriert, gewaschen und getrocknet. Das resultierende Produkt mit einem Schmelzbereich von 119 bis 122 °C entspricht in seiner Zusammensetzung dem Di-N,N'-oxydiäthylenthiuramhexasulfid.

Analyse : Di-N,N'-oxydiäthylenthiuramhexasulfid $C_{10}H_{16}N_2O_2S_8$
ber. : C 26,53 % H 3,56 % N 6,19 % S 56,65 %
gef. : C 26,3 % H 3,5 % N 6,0 % S 56,9 %

Die Ausbeute beträgt 84,0 g (92,8 % d. Th.).

Beispiel 7 (Vergleichsbeispiel)

Es wird wie in Beispiel 6 gearbeitet, aber ohne den Zusatz von Triäthylamin. Die Sauerstoffaufnahme erfolgt unter diesen Bedingungen nur sehr langsam ; nach 270 min. wird ein Reaktionsgemisch erhalten, das im wesentlichen die einzelnen Ausgangskomponenten in unveränderter Form bzw. als Dithiocarbamate enthält.

Beispiel 8

In der in Beispiel 1 beschriebenen Reaktionsapparatur werden 34,8 g (0,4 mol) Morpholin, 31,2 g (0,41 mol) Schwefelkohlenstoff, 24,2 g (0,41 mol) Trimethylamin und 12,8 g (0,4 mol) Schwefel in 300 ml Isopropanol mit Sauerstoff zur Reaktiongebracht, wobei als Katalysator $0.2 \cdot 10^{-3}$ mol Mangan-II-acetat

eingesetzt werden. Die Reaktionstemperatur beträgt 60 °C, der Sauerstoffdruck, 1,8 bar. Nach 90 min. ist die Reaktion beendet. Durch Abfiltrieren und Waschen des gebildeten Niederschlags werden 73,1 g Di-N,N'-oxydiäthylenthiuramtetrasulfid mit einem Schmelzbereich 120-130 °C erhalten (94,1 % d. Th.).

## Beispiele 9-12

Es wird wie in Beispiel 1 gearbeitet, jedoch mit anderen Katalysatoren und bei unterschiedlichen Reaktionstemperaturen. Die entsprechenden Reaktionszeiten und Produktausbeuten sind in Tabelle 1 zusammengestellt.

Tabelle 1

| Beispiel | Katalysator (mmol) | | Reaktions-temp. (°C) | Reaktions-zeit (min.) | Ausbeute (% d. Th.) |
|---|---|---|---|---|---|
| 9 | CuCl | (0,1) | 50 | 110 | 96,9 |
| 10 | $CuCl_2$ | (0,05) | 65 | 95 | 96,2 |
| 11 | $Ce/NO_3)_3$ | (0,04) | 40 | 60 | 97,6 |
| 12 | $MnSO_4$ | (0,02) | 50 | 55 | 98,8 |

## Beispiele 13-16

Es wird wie in Beispiel 1 gearbeitet, jedoch werden andere Lösungsmittel eingesetzt. Die Ergebnisse sind in der Tabelle 2 zusammengefaßt.

Tabelle 2

| Beispiel | Lösungsmittel (300 ml) | Reaktionszeit (min.) | Ausbeute (% d. Th.) |
|---|---|---|---|
| 13 | Toluol | 190 | 95,7 |
| 14 | Isopropanol | 75 | 99,2 |
| 15 | Äthanol | 65 | 98,5 |
| 16 | Methanol/10 % Wasser | 60 | 97,8 |

## Beispiel 17

Im folgenden Beispiel wird als sauerstoffhaltiges Gas Luft verwendet. In der in Beispiel 1 beschriebenen Weise werden 34,5 g (0,4 mol) Piperidin, 31,2 g (0,41 mol) Schwefelkohlenstoff, 9,9 g (0,1 mol) Triäthylamin und 12,8 g (0,4 Grammatom) Schwefel in 300 ml Methanol bei Gegenwart von $0{,}025 \cdot 10^{-3}$ mol Mangan-II-acetat und Luft (Gesamtdruck 5 bar) zur Reaktion gebracht. Die Reaktionstemperatur beträgt 50 °C, die Reaktionszeit 120 min. Dabei wird das Dipentamethylenthiurampolysulfid in einer Ausbeute von 75,2 g erhalten (97,9 % d. Th.).

## Beispiele 18-21

In den folgenden Beispielen werden andere sekundäre Amine eingesetzt. Die Versuchsdurchführung erfolgt jeweils in der in Beispiel 1 beschriebenen Weise. Als Lösungsmittel werden jeweils 300 ml Methanol verwendet, der Sauerstoffdruck beträgt 1,7 bar.

Die weiteren Reaktionsbedingungen sowie die Ausbeuten und Schmelzpunkte der entsprechenden Thiurampolysulfide sind in der folgenden Tabelle 3 zusammengefaßt.

(Siehe Tabelle 3 Seite 8 f.)

Tabelle 3

| Beispiel | sek. Amin (mol) | $CS_2$/$NET_3$/Schwefel (mol/mol/Grammatom) | Mn-II-acetat (mmol) | Reaktions-temperatur (°C) | Reaktions-zeit (min.) | Produkt Fp (°C) | Ausbeute (% d. Th.) |
|---|---|---|---|---|---|---|---|
| 18 | $(CH_3)_2NH$ (0,4) | 0,41/0,05/0,8 | 0,05 | 50 | 40 | $((CH_3)_2NCS_2)_2S_4$ 99 - 104 | 98,0 |
| 19 | $(CH_3)_2NH$ (0,5) | 0,51/0,05/0,5 | 0,05 | 60 | 75 | $((CH_3)_2NCS_2)_2S_2$ 110 - 126 | 97,7 |
| 20 | $(C_2H_5)NH$ (0,3) | 0,31/0,1/0,3 | 0,2 | 30 | 125 | $((C_2H_5)_2NCS_2)_2S_2$ wachsartig | 93,8 |
| 21 | $(CH_2)_4NH$ (0,4) | 0,41/0,1/0,8 | 0,05 | 50 | 55 | $((CH_2)_4NCS_2)_2S_4$ 133 - 136 | 92,8 |

**Patentansprüche**

1. Verfahren zur Herstellung von mit aliphatischen, araliphatischen, cycloaliphatischen und/oder aromatischen Kohlenwasserstoffresten substituierten Thiurampolysulfiden, ausgehend von einem entsprechend substituierten sekundären Amin, Schwefelkohlenstoff und einem Oxidationsmittel, dadurch gekennzeichnet, daß man als weitere Ausgangsverbindung Schwefel einsetzt, das Verfahren in Gegenwart eines tertiären Amins und eines ein Metall der Nebengruppenelemente enthaltenden Katalysators bei Temperaturen von 0 bis 150 °C durchführt und als Oxidationsmittel Sauerstoff oder ein sauerstoffhaltiges Gas verwendet.

2. Verfahren nach Anspruch 1, dadurch gekennzeichnet, daß pro Mol sekundäres Amin 1 bis 1,2 Mol Schwefelkohlenstoff eingesetzt werden.

3. Verfahren nach den Ansprüchen 1 und 2, dadurch gekennzeichnet, daß pro Mol sekundäres Amin 1 bis 3 Grammatom Schwefel eingesetzt werden.

4. Verfahren nach den Ansprüchen 1 bis 3, dadurch gekennzeichnet, daß der metallhaltige Katalysator in Mengen von 0,01 bis 5 mMol, bezogen auf ein Mol sekundäres Amin eingesetzt wird.

5. Verfahren nach den Ansprüchen 1 bis 4, dadurch gekennzeichnet, daß als Katalysator Cer, Mangan, Kupfer, Eisen, Kobalt, Molybdän oder Vanadin in elementarer Form, als Salze, Oxide, Komplexe oder organische Verbindungen oder ein Gemisch derselben eingesetzt wird.

6. Verfahren nach den Ansprüchen 1 bis 5, dadurch gekennzeichnet, daß ein tertiäres Amin mit einem pKa-Wert $> 8$ eingesetzt wird.

7. Verfahren nach den Ansprüchen 1 bis 6, dadurch gekennzeichnet, daß als Lösungsmittel ein gegebenenfalls substituierter aromatischer Kohlenwasserstoff, ein niederer Alkohol mit bis zu 6 C-Atomen, ein Gemisch dieser Lösungsmittel oder ein Gemisch des niederen Alkohols mit Wasser eingesetzt wird.

8. Verfahren nach den Ansprüchen 1 bis 7, dadurch gekennzeichnet, daß die Reaktion bei Temperaturen von 20 bis 90 °C durchgeführt wird.

**Claims**

1. A process for the production of thiuram polysulfides substituted by aliphatic, araliphatic, cycloaliphatic and/or aromatic hydrocarbon radicals from a correspondingly substituted secondary amine, carbon disulfide and an oxidizing agent, characterized in that sulfur is used as an additional starting compound, the process is carried out in the presence of a tertiary amine and a catalyst containing a metal of the secondary group elements at temperatures of 0 to 150 °C and oxygen or an oxygen-containing gas is used as the oxidizing agent.

2. A process as claimed in claim 1, characterized in that 1 to 1.2 mols carbon disulfide is used per mol secondary amine.

3. A process as claimed in claims 1 and 2, characterized in that 1 to 3 gram atoms sulfur are used per mol secondary amine.

4. A process as claimed in claims 1 to 3, characterized in that the metal-containing catalyst is used in quantities of from 0.01 to 5 mmols per mol secondary amine.

5. A process as claimed in claims 1 to 4, characterized in that cerium, manganese, copper, iron, cobalt, molybdenum or vanadium in elemental form, as a salt, oxide, complex or organic compound, or a mixture thereof, is used as the catalyst.

6. A process as claimed in claims 1 to 5, characterized in that a tertiary amine having a pKa value of $> 8$ is used.

7. A process as claimed in claims 1 to 6, characterized in that the solvent used is an optionally substituted aromatic hydrocarbon, a lower alcohol containing up to 6 C atoms, a mixture of these solvents or a mixture of the lower alcohol with water.

8. A process as claimed in claims 1 to 7, characterized in that the reaction is carried out at temperatures of 20 to 90 °C.

**Revendications**

1. Procédé de préparation de polysulfures de thiurame, substitués par des radicaux hydrocarbonés aliphatiques, araliphatiques, cycloaliphatiques et/ou aromatiques, à partir d'une amine secondaire substituée de façon correspondante, de sulfure de carbone et d'un oxydant, caractérisé en ce que l'on utilise du soufre comme autre produit de départ, en ce que l'on met en œuvre le procédé en présence d'une amine tertiaire et d'un catalyseur contenant un métal faisant partie des éléments de transition, à des températures de 0 à 150 °C, et en ce que l'on utilise, comme oxydant, de l'oxygène ou un gaz contenant de l'oxygène.

2. Procédé conforme à la revendication 1, caractérisé en ce que l'on utilise 1 à 1,2 mole de sulfure de carbone par mole d'amine secondaire.

3. Procédé conforme aux revendications 1 et 2, caractérisé en ce que l'on utilise 1 à 3 atomes-grammes de soufre par mole d'amine secondaire.

4. Procédé conforme aux revendications 1 à 3, caractérisé en ce que l'on utilise le catalyseur contenant un métal en des quantités de 0,01 à 5 millimoles, pour 1 mole d'amine secondaire.

5. Procédé conforme aux revendications 1 à 4, caractérisé en ce que l'on utilise, comme catalyseur, du cérium, du manganèse, du cuivre, du fer, du cobalt, du molybdène ou du vanadium, sous forme élémentaire ou sous forme de sels, d'oxydes, de complexes ou de composés organiques, ou un mélange de ces corps.

6. Procédé conforme aux revendications 1 à 5, caractérisé en ce que l'on utilise une amine tertiaire présentant une valeur de pKa supérieure à 8.

7. Procédé conforme aux revendications 1 à 6, caractérisé en ce que l'on utilise, comme solvant, un hydrocarbure aromatique éventuellement substitué, un alcool inférieur comportant jusqu'à 6 atomes de carbone, un mélange de ces solvants, ou un mélange d'un alcool inférieur avec de l'eau.

8. Procédé conforme aux revendications 1 à 7, caractérisé en ce que la réaction est effectuée à des températures de 20 à 90 °C.